# EUROPEAN PATENT SPECIFICATION

(11) **EP 4 473 932 B1**
(45) Date of publication and mention of the grant of the patent: **18.11.2026**
(21) Application number: 24206497.0
(22) Date of filing: 27.09.2018
(51) Int. Cl.: A61B 34/30, A61B 90/00

(54) **ROBOTIC SURGICAL DEVICES WITH TRACKING CAMERA TECHNOLOGY AND RELATED SYSTEMS**
ROBOTISCHE CHIRURGISCHE VORRICHTUNGEN MIT VERFOLGUNGSKAMERATECHNOLOGIE UND VERWANDTE SYSTEME
DISPOSITIFS CHIRURGICAUX ROBOTISÉS AVEC TECHNOLOGIE DE CAMÉRA DE SUIVI ET SYSTÈMES ASSOCIÉS

(30) Priority: 27.09.2017 US 201762564076 P
(43) Date of publication of application: 11.12.2024
(62) Document number(s) of the earlier application(s) in accordance with Art. 76 EPC: 18863267.3 / 3 687 370
(73) Proprietor: Virtual Incision Corporation, Lincoln, NE 68512 (US)
(72) Inventor: FARRITOR, Shane, Lincoln, NE 68526 (US); OLEYNIKOV, Dmitry, Omaha, NE 68164 (US); WOOD, Nathan, Lincoln, NE 68506 (US); DUMPERT, Jason, Omaha, NE 68135 (US); REICHENBACH, Mark, Lincoln, NE 68516 (US); CUBRICH, Lou, Lincoln, NE 68503 (US)
(74) Representative: Bradbury, Simon Timothy Nicholas

(56) References cited:
- WO-A1-2010/039394
- WO-A1-2015/031777
- US-A1- 2009 326 322
- US-A1- 2017 007 336

## Description

### Cross-Reference to Related Application(s)

This application claims the benefit under 35 U.S.C. § 119(e) to U.S. Provisional Application 62/564,076, filed September 27, 2017 and entitled "Robotic Surgical Devices with Camera Tracking and Related Systems and Methods".

### Field of the Invention

The implementations disclosed herein relate to various medical devices and related components, including robotic and/or in vivo medical devices and related components. Certain implementations include various robotic medical devices, including robotic devices that are disposed within a body cavity and positioned using a support component disposed through an orifice or opening in the body cavity and further including a camera that is positioned through the support component and can be operated to manually or automatically track the arms or end effectors of the robotic device.

### Background of the Invention

Invasive surgical procedures are essential for addressing various medical conditions. When possible, minimally invasive procedures such as laparoscopy are preferred.

However, known minimally invasive technologies such as laparoscopy are limited in scope and complexity due in part to 1) mobility restrictions resulting from using rigid tools inserted through access ports, and 2) limited visual feedback. Known robotic systems such as the *da Vinci*^{®} Surgical System (available from Intuitive Surgical, Inc., located in Sunnyvale, CA) are also restricted by the access ports, as well as having the additional disadvantages of being very large, very expensive, unavailable in most hospitals, and having limited sensory and mobility capabilities.

There is a need in the art for improved surgical methods, systems, and devices.
US 20090326322 A1 discloses a medical robotic system including an entry guide with articulatable instruments such as surgical tools and a camera extending out of its distal end. The camera instrument is manipulatable by a camera manipulator, which has a first mechanism for pivoting a focal point of the camera instrument about a pivot of the camera instrument and a second mechanism for positioning the pivot within a three-dimensional space in response to translational commands received from one or a coupled pair of input devices. The system also includes a controller which is configured to receive sensed movement of the input devices, and cause actuation of the first mechanism in response to the sensed movement if the system is in an orientational mode and cause actuation of the second mechanism in response to the sensed movement if the system is in a translational mode.

### Brief Summary of the Invention

Discussed herein are various robotic surgical systems, including certain systems having camera lumens constructed and arranged to receive various camera systems, including tracking camera systems. Further implementations relate to surgical insertion devices constructed and arranged to be used to insert various surgical devices into a cavity of a patient while maintaining insufflation of the cavity.

According to an aspect of the present disclosure, there is provided a robotic surgical system of Claim 1. Optional features of the robotic surgical system are provided in dependent Claims 2 to 11.

While multiple implementations are disclosed, still other implementations of the present invention will become apparent to those skilled in the art from the following detailed description, which shows and describes illustrative implementations of the invention. As will be realized, the invention is capable of modifications in various obvious aspects, all without departing from the scope of the present invention. Accordingly, the drawings and detailed description are to be regarded as illustrative in nature and not restrictive.

### Brief Description of the Drawings

FIG. 1A is a side view schematic view of the robotic surgical system, according to one embodiment.
FIG. 1B is a front view of the robotic surgical system showing the robotic device with an engaged positionable camera, according to one embodiment.
FIG. 2A is three-quarters front view of the robotic device with an engaged positionable camera, according to one embodiment.
FIG. 2B is a three-quarters perspective view of the robot of the implementation of FIG. 2 without the camera.
FIG. 2C is a three-quarters perspective view of the camera of the implementation of FIG. 2 without the robot.
FIG. 3A is a close-up three-quarters front view of the robotic device with an engaged positionable camera, according to one embodiment.
FIG. 3B is a close-up three-quarters front view of the robotic device with an engaged positionable camera showing the degrees of freedom of the arms, according to one embodiment.
FIG. 4A is a perspective view of a surgical device showing various workspaces for one arm, according to one embodiment.
FIG. 4B is a further perspective view of the surgical device of FIG. 6A, showing the workspace of the other arm.
FIG. 5 is a perspective view of a surgical device showing various workspaces for the arms, according to one embodiment.
FIG. 6 is a further perspective view of the surgical device of FIG. 6A, showing the workspace of one arm.
FIG. 7 is a zoomed in view of the camera operations system showing the components on the camera handle, according to one embodiment.
FIG. 8A is perspective three quarters view of the surgical robotic device and positionable camera showing the camera field of view, according to one implementation.
FIG. 8B is a cutaway side view of the robotic surgical device comprising a positionable camera and showing a first degree of freedom, according to one embodiment.
FIG. 8C is a cutaway side view of the robotic surgical device comprising a positionable camera and showing a second degree of freedom, according to one embodiment.
FIG. 8D is a perspective three-quarters side view of the robotic surgical device comprising a positionable camera and showing coordinate reference fames, according to the present invention.
FIG. 8E is a view from the perspective of the positionable camera inserted into the robotic surgical device and showing the end effectors within that field of view.
FIG. 8F is a view from the perspective of the positionable camera inserted into the robotic surgical device and showing the locating of the end effectors within that field of view and generating an origin, according to the present invention.
FIG. 8G is a perspective three-quarters side view of the robotic surgical device comprising a positionable camera and showing coordinate reference fames and the generation of midpoint calculations, according to the present invention.
FIG. 8H is a view from the perspective of the positionable camera inserted into the robotic surgical device and showing the end effectors within that field of view and midpoint, according to the present invention.
FIG. 8I is a view from the perspective of the positionable camera inserted into the robotic surgical device and showing the re-positioning of the camera, according to the present invention.
FIG. 9 is a front view of the robotic surgical system showing the robotic device with an engaged positionable camera, according to one embodiment.
FIG. 10A is a perspective view of the surgical console, according to one implementation.
FIG. 10B is a perspective view of the surgical console, according to another implementation.
FIG. 11A is a schematic view of the robot, pod and console, showing the schematic connection maps between the components, according to one implementation.
FIG. 11B is a perspective pop-out view of the interface pod on the support cart, according to one implementation.
FIG. 12 is a top view of several surgical tools, according to certain embodiments.
FIG. 13 is a perspective top view showing the installation of the surgical tools into the arms, according to one implementation.
FIG. 14 is a perspective top view showing the surgical robotic device showing the sleeves, according to one implementation.
FIG. 15 is a front view of the robotic surgical system affixed via a clamp attached to a support arm, according to one implementation.
FIG. 16 is a close-up perspective view of the robotic clamp, according to one implementation.

### Detailed Description

The various systems and devices disclosed herein relate to devices for use in medical procedures and systems. More specifically, various implementations relate to various medical devices, including robotic devices having tracking camera systems and related methods and systems, including, in some implementations, controlling consoles and other devices to provide complete systems.

It is understood that the various implementations of robotic devices and related methods and systems disclosed herein can be incorporated into or used with any other known medical devices, systems, and methods. For example, the various implementations disclosed herein may be incorporated into or used with any of the medical devices and systems disclosed in U.S. Patents 7,492,116 (filed on October 31, 2007 and entitled "Robot for Surgical Applications"), 7,772,796 (filed on April 3, 2007 and entitled "Robot for Surgical Applications"), 8,179,073 (issued May 15, 2011, and entitled "Robotic Devices with Agent Delivery Components and Related Methods"), 8,343,171 (issued January 1, 2013 and entitled "Methods and Systems of Actuation in Robotic Devices"), 8,679,096 (issued March 25, 2014 and entitled "Multifunctional Operational Component for Robotic Devices"), 8,834,488 (issued September 16, 2014 and entitled "Magnetically Coupleable Surgical Robotic Devices and Related Methods"), 8,894,633 (issued November 25, 2014 and entitled "Modular and Cooperative Medical Devices and Related Systems and Methods"), 8,968,267 (issued March 3, 2015 and entitled "Methods and Systems for Handling or Delivering Materials for Natural Orifice Surgery"), 8,968,332 (issued March 3, 2015 and entitled "Magnetically Coupleable Robotic Devices and Related Methods"), 8,974,440 (issued March 10, 2015 and entitled "Modular and Cooperative Medical Devices and Related Systems and Methods"), 9,010,214 (April 21, 2015 and entitled "Local Control Robotic Surgical Devices and Related Methods"), 9,060,781 (issued June 23, 2015 and entitled "Methods, Systems, and Devices Relating to Surgical End Effectors" ), 9,089,353 (issued July 28, 2015 and entitled "Robotic Surgical Devices, Systems, and Related Methods"), 9,498,292 (issued November 22, 2016 and entitled "Single Site Robotic Devices and Related Systems and Methods"), 9,579,088 (issued February 28, 2017 and entitled "Methods, Systems, and Devices for Surgical Visualization and Device Manipulation"), 9,743,987 (August 29, 2017 and entitled "Methods, Systems, and Devices Relating to Robotic Surgical Devices, End Effectors, and Controllers"), 9,770,305 (issued September 26, 2017 and entitled "Robotic Surgical Devices, Systems, and Related Methods"), and 9,888,966 (issued February 13, 2018 and entitled "Methods, Systems, and Devices Relating to Force Control Surgical Systems).

Further, the various implementations disclosed herein may be incorporated into or used with any of the medical devices and systems disclosed in copending U.S. Published Applications 2014/0046340 (filed March 15, 2013 and entitled "Robotic Surgical Devices, Systems, and Related Methods"), 2014/0058205 (filed January 10, 2013 and entitled "Methods, Systems, and Devices for Surgical Access and Insertion"), 2014/0303434 (filed March 14, 2014 and entitled "Robotic Surgical Devices, Systems, and Related Methods"), 2015/0051446 (filed July 17, 2014 and entitled "Robotic Surgical Devices, Systems, and Related Methods"), 2016/0074120 (filed September 14, 2015, and entitled "Quick-Release End Effectors and Related Systems and Methods"), 2016/0135898 (filed November 11, 2015 entitled "Robotic Device with Compact Joint Design and Related Systems and Methods"), 2016/0157709 (filed February 8, 2016 and entitled "Medical Inflation, Attachment, and Delivery Devices and Related Methods"), 2017/0035526 (filed August 3, 2016 and entitled "Robotic Surgical Devices, Systems, and Related Methods"), 2017/0354470 (filed May 18, 2017 and entitled "Robotic Surgical Devices, Systems, and Related Methods"), 2018/0055584 (filed August 30, 2017 and entitled "Robotic Device with Compact Joint Design and an Additional Degree of Freedom and Related Systems and Methods"), 2018/0056527 (filed August 25, 2017 and entitled "Quick-Release End Effector Tool Interface"), 2018/0140377 (filed November 22, 2017 and entitled "Gross Positioning Device and Related Systems and Methods"), 2018/0147019 (filed November 29, 2017 and entitled "User Controller with User Presence Detection and Related Systems and Methods"), and 2018/0161122 (filed December 14, 2017 and entitled "Releasable Attachment Device for Coupling to Medical Devices and Related Systems and Methods"), In addition, the various implementations disclosed herein may be incorporated into or used with any of the medical devices and systems disclosed in U.S. Application 62/614,127 (filed January 5, 2018).

Certain device and system implementations disclosed in the patents and/or applications listed above can be positioned within a body cavity of a patient in combination with a support component similar to those disclosed herein. An "in vivo device" as used herein means any device that can be positioned, operated, or controlled at least in part by a user while being positioned within a body cavity of a patient, including any device that is coupled to a support component such as a rod or other such component that is disposed through an opening or orifice of the body cavity, also including any device positioned substantially against or adjacent to a wall of a body cavity of a patient, further including any such device that is internally actuated (having no external source of motive force), and additionally including any device that may be used laparoscopically or endoscopically during a surgical procedure. As used herein, the terms "robot," and "robotic device" shall refer to any device that can perform a task either automatically or in response to a command.

Certain implementations provide for insertion of the present invention into the cavity while maintaining sufficient insufflation of the cavity. Further implementations minimize the physical contact of the surgeon or surgical users with the present invention during the insertion process. Other implementations enhance the safety of the insertion process for the patient and the present invention. For example, some implementations provide visualization of the present invention as it is being inserted into the patient's cavity to ensure that no damaging contact occurs between the system/device and the patient. In addition, certain implementations allow for minimization of the incision size/length. Further implementations reduce the complexity of the access/insertion procedure and/or the steps required for the procedure. Other implementations relate to devices that have minimal profiles, minimal size, or are generally minimal in function and appearance to enhance ease of handling and use.

As in manual laparoscopic procedures, a known insufflation system can be used to pump sterile carbon dioxide (or other gas) into the patient's abdominal cavity. This lifts the abdominal wall from the organs and creates space for the robot. In certain implementations, the system has no direct interface with the insufflation system. Alternatively, the system can have a direct interface to the insufflation system.

In certain implementations, the insertion port is a known, commercially-available flexible membrane placed transabdominally to seal and protect the abdominal incision. This off-the-shelf component is the same device used in the same way for Hand-Assisted Laparoscopic Surgery (HALS). The only difference is that the working arms of the robot are inserted into the abdominal cavity through the insertion port rather than the surgeon's hand. The robot body seals against the insertion port, thereby maintaining insufflation pressure. The port is single-use and disposable. Alternatively, any known port can be used.

Certain implementations disclosed herein relate to "combination" or "modular" medical devices that can be assembled in a variety of configurations. For purposes of this application, both "combination device" and "modular device" shall mean any medical device having modular or interchangeable components that can be arranged in a variety of different configurations, and the related systems. The modular components and combination devices disclosed herein also include segmented triangular or quadrangular-shaped combination devices. These devices, which are made up of modular components (also referred to herein as "segments") that are connected to create the triangular or quadrangular configuration, can provide leverage and/or stability during use while also providing for substantial payload space within the device that can be used for larger components or more operational components. As with the various combination devices disclosed and discussed above, according to one implementation these triangular or quadrangular devices can be positioned inside the body cavity of a patient in the same fashion as those devices discussed and disclosed above.

The various system implementations described herein are used to perform robotic surgery. Further, the various implementations disclosed herein can be used in a minimally invasive approach to a variety of procedures that are typically performed "open" by known technologies, with the potential to improve clinical outcomes and health care costs, including, for example, general surgery applications in the abdominal cavity, such as, for example, colon resection and other known procedures. Further, the various implementations disclosed herein can be used in place of the known mainframe-like laparoscopic surgical robots that reach into the body from outside the patient. That is, the less-invasive robotic systems, methods, and devices according to the implementations disclosed herein feature small, self-contained surgical devices that are inserted in their entireties through a single incision in the patient's abdomen. Designed to utilize existing tools and techniques familiar to surgeons, the devices disclosed herein will not require a dedicated operating room or specialized infrastructure, and, because of their much smaller size, are expected to be significantly less expensive than existing robotic alternatives for laparoscopic surgery. Due to these technological advances, the various implementations herein could enable a minimally invasive approach to procedures performed in open surgery today. In certain implementations, the various systems described herein are based on and/or utilize techniques used in manual laparoscopic surgery including insufflation of the abdominal cavity and the use of ports to insert tools into the abdominal cavity.

As will be described in additional detail below, components of the various system implementations disclosed or contemplated herein include a control console and a robot having a tracking camera system. The robot implementations are constructed and arranged to be inserted into the insufflated abdominal cavity. The tracking camera system can be an integrated camera system that captures a view of the surgical target and can be manually or automatically controlled to track and capture an ongoing view of the arms and/or end effectors of the robotic device. The surgeon can then use that view on a display to help control the robot's movements. In certain implementations, the camera is designed so that it can be removed so it can be cleaned and used in other applications.

In other implementations as will be discussed in further detail herein, the system can include disposable or permanent sleeves positioned on or attached to the robotic device, an electro-surgery cautery generator, an insertion port, a support arm/structure, a camera, remote surgical displays, end-effectors (tools), an interface pod, a light source, and other system components.

The various implementations are disclosed in additional detail in the attached figures, which may include some written description therein.

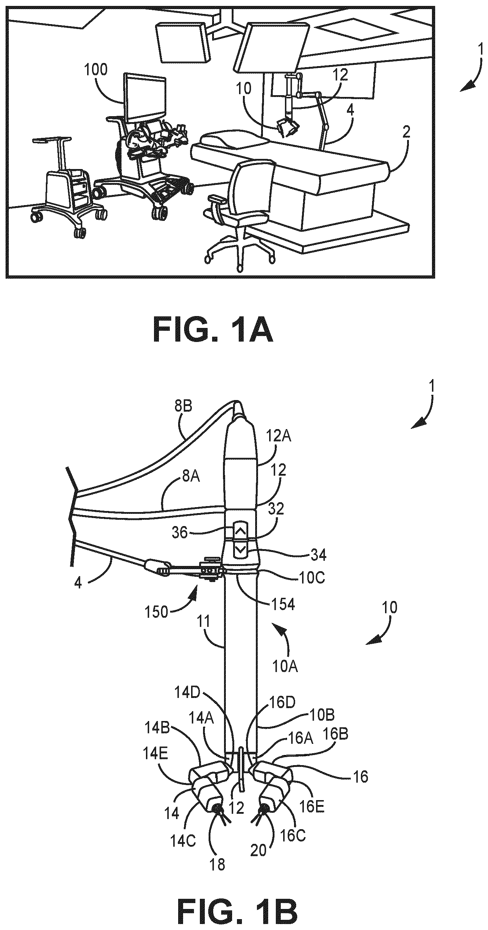

According to one implementation, the Robotically Assisted Surgical Device (RASD) system 1 has several components. In one such implementation, and as shown in FIG. 1A and FIG. 1B, a surgical robotic device 10 having a robotically articulated camera 12 disposed therein and an external surgeon control console 100 is provided. In the implementation of FIG. 1A, the robotic device 10 and the camera 12 are shown mounted to the operating table 2 using a robot support arm 4, in accordance with one implementation. The system 1 can be, in certain implementations, operated by the surgeon and one surgical assistant.

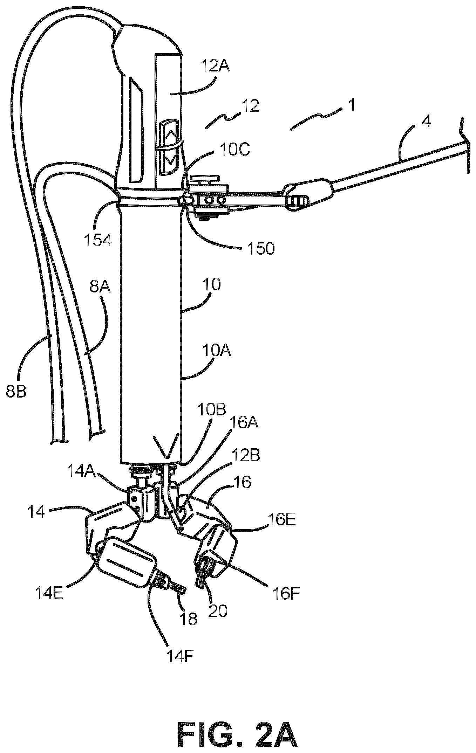

FIG. 1B and FIG. 2A depict exemplary implementations of the robotic device 10 having a body 10A (or torso) having a distal end 10B and proximal end 10C, with the camera 12 disposed therein, as has been previously described. Briefly, the robotic device 10 has two robotic arms 14, 16 operably coupled thereto and a camera component or "camera" 12 disposed between the two arms 14, 16 and positionable therein. That is, device 10 has a first (or "right") arm 14 and a second (or "left) arm 16, both of which are operably coupled to the device 10 as discussed in additional detail below. The device 10 as shown has a casing (also referred to as a "cover" or "enclosure") 11. The device 10 is also referred to as a "device body" 10A and has two rotatable cylindrical components (also referred to as "shoulders" or "turrets"): a first (or "right") shoulder 14A and a second (or "left") shoulder 16A. Each arm 14, 16 also has an upper arm (also referred to herein as an "inner arm," "inner arm assembly," "inner link," "inner link assembly," "upper arm assembly," "first link," or "first link assembly") 14B, 16B, and a forearm (also referred to herein as an "outer arm," "outer arm assembly," "outer link," "outer link assembly," "forearm assembly," "second link," or "second link assembly") 14C, 16C. The right upper arm 14B is operably coupled to the right shoulder 14A of the body 10A at the right shoulder joint 14D and the left upper arm 16B is operably coupled to the left shoulder 16A of the body 10 at the left shoulder joint 16D. Further, for each arm 14, 16, the forearm 14C, 16C is rotatably coupled to the upper arm 14B, 16B at the elbow joint 14E, 16E.

In various implementations, the device 10 and each of the links of the arms 14, 16 contain a variety of actuators or motors. In one embodiment, any of the motors discussed and depicted herein can be brush or brushless motors. Further, the motors can be, for example, 6 mm, 8 mm, or 10 mm diameter motors. Alternatively, any known size that can be integrated into a medical device can be used. In a further alternative, the actuators can be any known actuators used in medical devices to actuate movement or action of a component. Examples of motors that could be used for the motors described herein include the EC 10 BLDC + GP10A Planetary Gearhead, EC 8 BLDC + GP8A Planetary Gearhead, or EC 6 BLDC + GP6A Planetary Gearhead, all of which are commercially available from Maxon Motors, located in Fall River, MA. There are many ways to actuate these motions, such as with DC motors, AC motors, permanent magnet DC motors, brushless motors, pneumatics, cables to remote motors, hydraulics, and the like.

In these implementations, the robotic device 10 and camera 12 are both connected to the surgeon console using a cable: the robot cable 8A and camera cable 8B. Alternatively, any connection configuration can be used. In certain implementations, the system can also interact with other devices during use such as a electrosurgical generator, an insertion port, and auxiliary monitors.

As shown in FIG. 1B, the camera 12 comprises a camera latch 32 and insertion 34 and retraction 36 controls or buttons. The robotic device 10 is supported by a support arm 4 that is clamped to the operating table (shown in FIG. 1A at 2). In these implementations, a robot clamp 150 is used to connect the support arm 4 to an acceptance ring 11 on the robot handle or body 10A.

According to the implementations of FIG. 1B and FIG. 2A, the arms 14, 16 each have active degrees of freedom and an additional active joint 14F, 16F to actuate the end effectors, or tools 18, 20. It is understood that more or less degrees of freedom could be included. The device in this implementation has a connection line 8 (also referred to as a "pigtail cable") (partially shown) that includes electrical power, electrocautery, and information/communication signals. In certain implementations, the device has distributed control electronics and software to help control the device 10. Some buttons can be included to support insertion and extraction of the device into and out of the abdominal cavity. In this implementation, the integrated camera 12 is also shown inserted in the device body 10A. When inserted into the body 10A, the camera 12 has a handle or body 12A that extends proximally from the proximal body end 10C and a flexible camera imager 12B extending from the distal body end 10B.

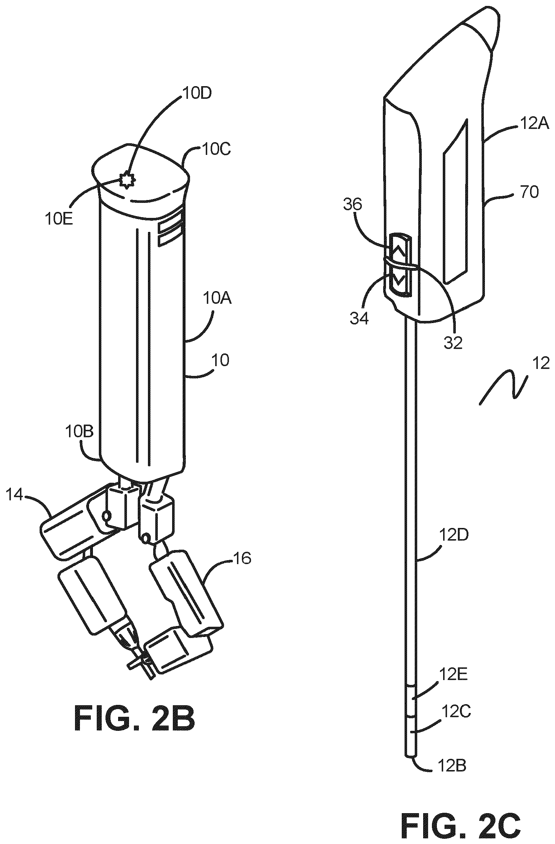

FIGS. 2B and 2C depict the robotic device 10 with the camera assembly 12 removed, according to one implementation. In these implementations, and as shown in FIG. 2 and FIGS. 3-4, the camera imager 12B is designed to be positioned between the two arms 14, 16 and capture that view between the two arms 14, 16. In these implementations, the camera 12 extends through the robot body 10A such that the camera imager 12B exits near the joints between the body and the robotic arms (the "shoulder" joints 14A, 16A). The camera 12 has a flexible, steerable tip 12C to allow the user to adjust the viewing direction. The end effectors 18, 20 on the distal end of the arms 14, 16 can include various tools 18, 20 (scissors, graspers, needle drivers and the like). In certain implementations, the tools 18, 20 are designed to be removable by a small twist of the tool knob that couples the end effector to the arm 14, 16.

As is shown in FIGS. 2B and 2C, the camera assembly 12 has a handle 12A and a long shaft 12D with the camera imager 12B at the distal tip 12C. In various implementations, the flexible tip 12C and therefore camera imager 12B can be steered or otherwise moved in two independent directions in relation to the shaft 12D at a flexible section 12E (black section on shaft) to change the direction of view. In certain implementations, the camera 12 has some control buttons 12F as shown. In some implementations, the camera assembly 12 can be used independently of the robotic device 10 as shown in FIG. 2C.

Alternatively, the assembly can be inserted into the robotic device 10 though a lumen 10D defined through the body 10A of the robotic device 10 as shown. In certain implementations, the lumen 10D includes a seal/port 10E to ensure that the patient's cavity remains insufflated (as shown in relation to FIG. 1B). According to one implementation, the robotic device 10 can have a sensor to determine if the camera is positioned in the camera lumen 10D of the device 10.

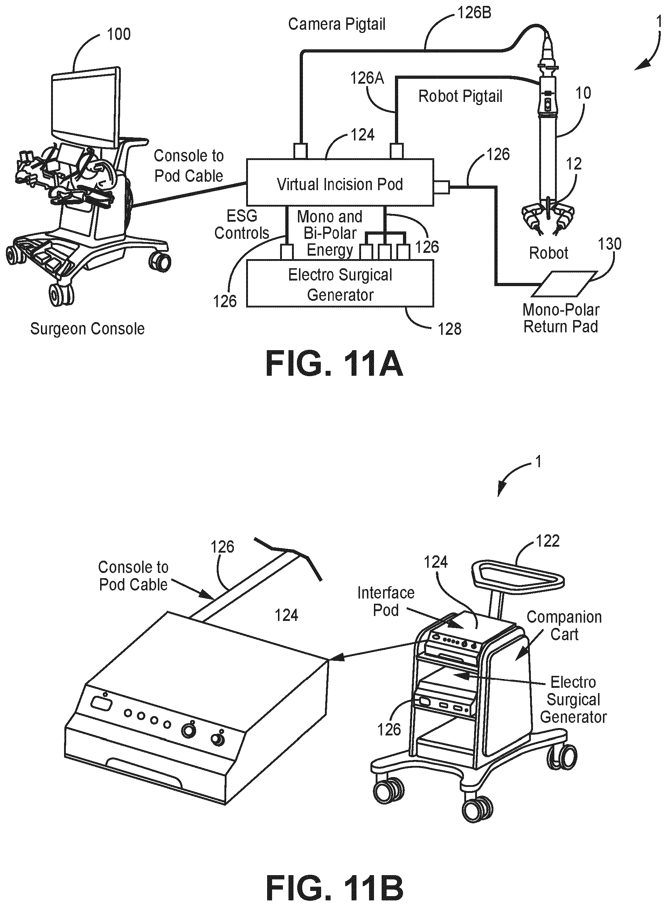

In use, the distal portion of the robotic device 10 is inserted inside the body of the patient. Thereafter, the robot and camera can both be controlled by the surgeon via the surgeon console sitting outside the sterile field. The surgeon console has user input devices (i.e. joysticks) that allow the surgeon to control the motion of the robot, as described in detail below. There are also pedal inputs and a touchscreen that control device 10 functions in certain implementations, as shown in FIGS. 11A-11B. The console can have a main display that provides images of the surgical environment via the robot camera.

It is understood that in the described implementations, the robotic device 10 has a pair of miniaturized human-like arms 14, 16 attached to a central body or handle 10A, as shown in FIG. 1B, FIG. 2A, FIG. 2B and FIG. 3A and FIG. 3B. Alternatively, any *in vivo* robot can be utilized with the system implementations disclosed or contemplated herein.

The robot handle 10A in the implementation of FIGS. 1B-3B has a lumen 10D (shown in FIG. 2B) and docking feature that allows the camera 12 to be inserted and removed from the body 10A while maintaining abdominal insufflation. When inserted (as shown in FIGS. 1B and 2A), the camera 12 has an articulating tip 12B that can include a light source and allows the surgeon to view the surgical tools 14, 16 18, 20 and surgical environment.

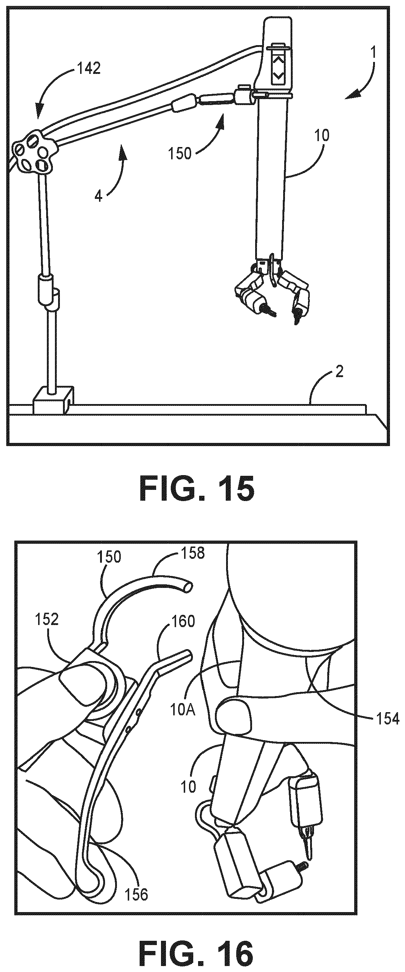

In these implementations, the camera 12 can be locked in place and can be removed using a latch button 32 on the camera handle 12A or elsewhere. In these implementations, the surgical robotic device is supported by a support arm 4 that is clamped to the operating table 2. As described in relation to FIGS. 15 and 16, a robot clamp is used to connect the support arm to an acceptance ring on the robot handle. Alternatively, the robotic device 10 can be supported via any known support component.

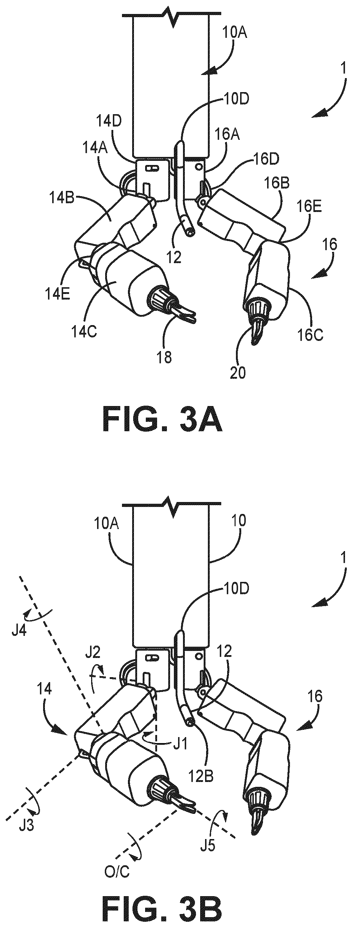

As shown in FIG. 3A and FIG. 3B, in use, after the camera 12 is inserted into the robot body 10A, the distal tip of the camera 12 passes through a lumen in the robot and extends into the surgical environment. The distal tip 12B of the camera 12 can then be actuated to provide views of the surgical tools and surgical target. It is understood that the camera 12 can be used with any similar robotic device having a camera lumen defined therethrough.

Each robot arm 14, 16 in this implementation has six degrees of freedom, including the open/close function of the tool, as shown in FIG. 3B. The robot shoulder is approximately a spherical joint similar to a human shoulder. The shoulder can yaw (J1), pitch (J2), and roll about the upper arm segment (J3). These first three axes of rotation roughly intersect at the shoulder joint. The robot elbow (J4) allows rotation of the forearm with respect to the upper arm. Finally, the tool can roll (J5) about the long axis of the tool and some tools have an open/close actuation function. In contrast, it is understood that a hook cautery tool does not open/close.

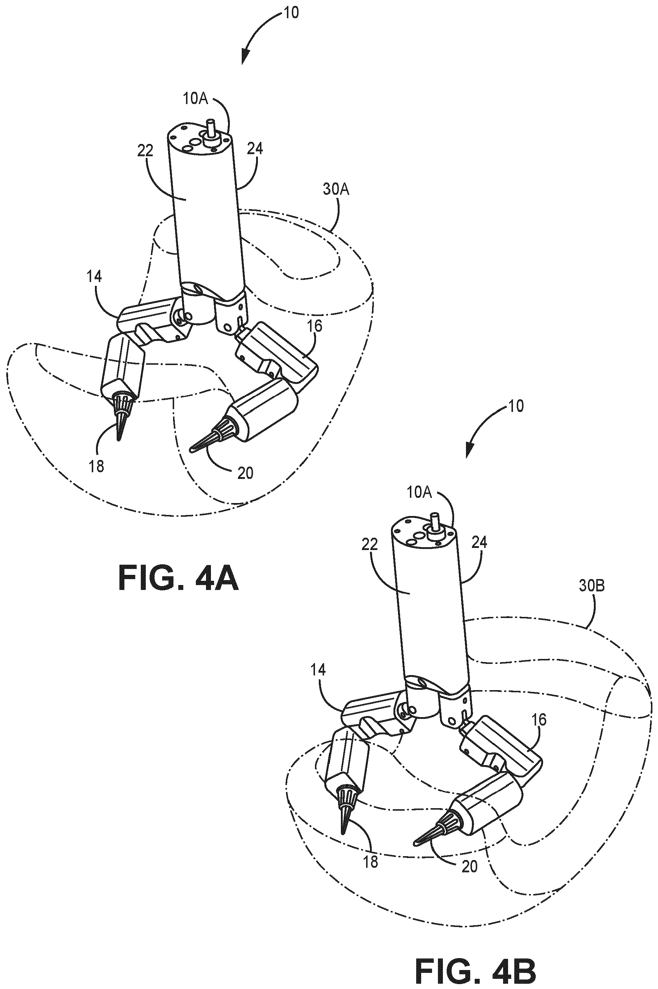

The surgical robot in this implementation has significant dexterity. As shown in FIG. 4A and FIG. 4B, the six degrees of freedom described above allow the robot's arms 14, 16 to reach into the confined spaces of the abdominal cavity.

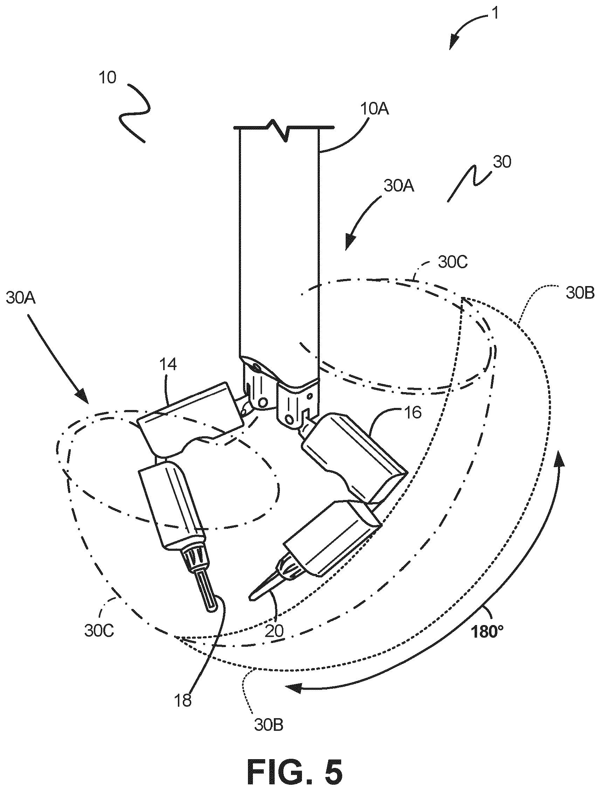

FIGS. 4A, 4B, 5 and schematically depict the entire workspace 30 as well as the individual reachable workspaces 30A, 30B of each of the arms 14, 16 of a robotic device 10, according to certain implementations. In these implementations, "workspace" 30 means the space 30 around the robotic device 10 in which either arm and / or end effector 18, 20 can move, access, and perform its function within that space.

FIG. 5 shows the regions that can be reached by the left arm and by the right arm. More specifically, FIG. 5 depicts a perspective view of the device body 10A and further schematically shows the entire workspace 30 as well as the individual workspaces 30A, 30B of the first arm 14 and second arm 16, respectively. Note that the each arm 14, 16 has a range of motion and corresponding workspace 30A, 30B that extends from the front 22 of the device to the back 24 of the device 10. Thus, the first arm 14 equally to the front 22 and the back 24, through about 180° of space relative to the axis of the device body 10A for each arm 14, 16. This workspace 30 allows the robotic device to work to the front 22 and back 24 equally well without having to reposition the body 10A. The overlap of these volumes represents a region that is reachable by both the left and right arms and is defined as the bi-manual robot workspace. The surgeon will have full robot dexterity when working in this bi-manual region.

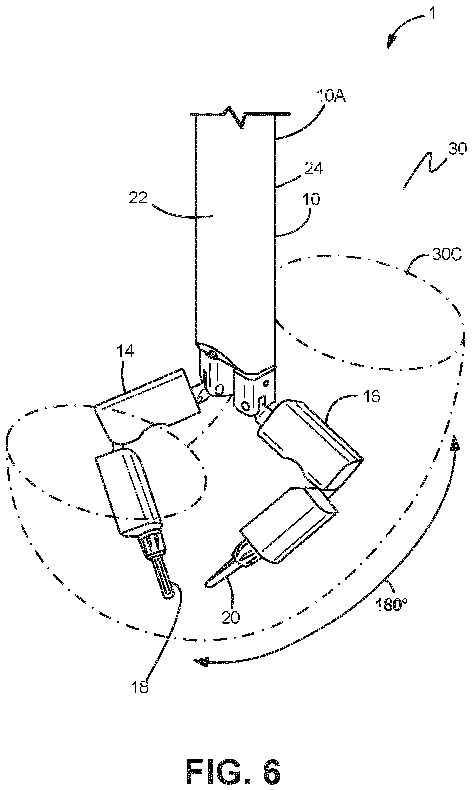

As best shown in FIG. 6, the overlap of the ranges of motion for the individual arms in these implementations also enables an intersecting, or bi-manual workspace 30C (as is also shown in FIG. 6A). It is understood that the intersecting workspace 30C in these implementations encompasses the workspace 30C reachable by both arms 14, 16 and end effectors 18, 20 in any individual device 10 position. Again, in these implementations, the intersecting workspace 30C includes a range of about 180° of space relative to the axis of the device body 10A.

The bi-manual workspace 30C is approximated by an ellipse that is rotated 180 degrees about the shoulder pitch joint (J2 in FIG. 3B) and is shown in FIG. 6. For one design, the ellipse is approximately 4.5" (11.5 cm) on the long axis and 3.25" (8.25 cm) on the minor axis. The bi-manual workspace 30 extends from in front of the robotic device 10 to below the robot and is also behind the back of the robot. This dexterity of the robotic arms 14, 16 allows the surgeon to operate the arms 14, 16 to work equally well anywhere inside this bi-manual workspace 30C.

In addition, according to this implementation, the surgical robotic device 10 can reach any area of the abdominal cavity because it can be easily repositioned during the procedure via "gross positioning." That is, the device 10 can be quickly, in a matter of seconds, be moved by adjusting the external support arm 4 and robot clamp 150. The combination of gross positioning of the robotic device 10 and the dexterity of the robot arms 14, 16 allow the surgeon to place the device 10 so it can work anywhere in the abdominal cavity with the arms 14, 16 well triangulated for the given procedure, as discussed below.

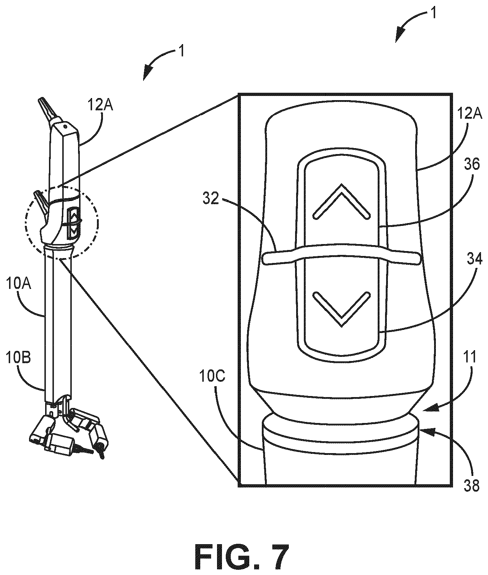

Turning to the insertion of the device 10 and camera 12 in greater detail, FIG. 7 depicts a detailed view of the handle 12A according to certain implementations. In FIG. 7, the camera 12 has a camera latch 32 and insertion 34 and retraction 36 controls or buttons. The robotic device 10 is supported by a support arm 4 that is clamped to the operating table (shown in FIG. 1A at 2). In these implementations, a robot clamp 150 is used to connect the support arm 4 to an acceptance ring 154 on the robot handle or body 10A.

In various implementations of the system 1, the device 10 is inserted into the abdomen of the patient by executing a series of configurations and / or arm positions. In certain implementations, the insertion 34 and retraction 36 controls or buttons allow the physician or user to executed the respective insertion and retraction steps / positions through the insertion and / or retraction, as would be understood. Further, in certain implementations, the camera latch 32 toggles the internal components of the device 10 and / or camera 12 into "locked" or "unlocked" positions, thereby securing the camera 12 within the device 10 or allowing it to be freely removed from the camera lumen, as would be understood.

Various implementations of the surgical robotic device 10 according to these implementations have an indicator light 38 or lights 38 disposed at the proximal end 10C of the device 10 and constructed and arranged to indicate any state of the device and can be any color or any intensity or of varying intensity. In certain implementations, LED lights or similar lighting components can be used, as would be appreciated by those of skill in the art.

In various implementations, the robotically articulated camera 12 is part of a system 1 to provide visual feedback to the surgeon from the perspective of the camera 12. In one specific implementation, the camera provides 1080p 60 Hz. digital video. Alternatively, the camera can provide any known video quality.

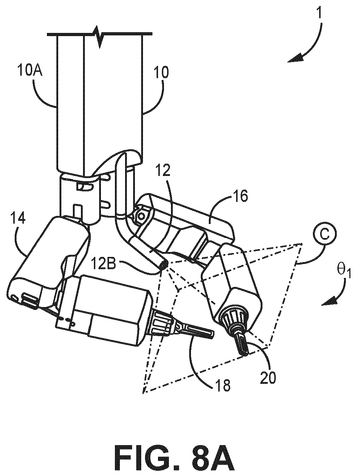

As is shown in the implementation of FIG. 8A, the camera 12 is constructed and arranged to be inserted into a lumen in the robot base link as shown in FIG. 1 so that the tip 12B of the camera is always positioned between the two robot arms 14, 16, and that the camera 12 has a field of view (shown with reference letter C in FIG. 8A).

It is likewise understood that when the robotic device 10 is repositioned during surgery, the camera 12 and robotic device 10 can move together or in a coordinated fashion in this configuration. This results in coordinated triangulation between the robot and tools 18, 20 for any configuration, positioning, and use of the device 10.

In accordance with certain implementations, the camera 12 is designed to visualize all possible positions of the robot's tools 18, 20. Accordingly, the camera tip 12B can be robotically articulated as to reposition the field of view (C). It is understood that in certain implementations, the surgeon controls this movement via the surgeon console 100 (described in detail in relation to FIG. 11A and FIG. 11B).

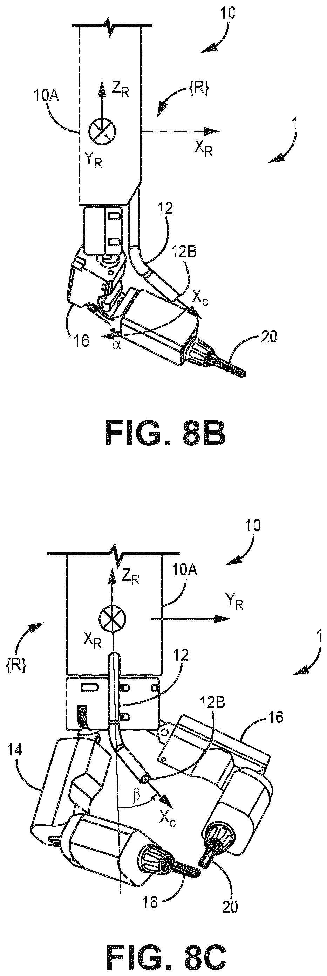

As shown in the implementations of FIG. 8B and FIG. 8C, the camera 12 can move in pitch (screen up/down) and / or yaw (screen left/right), respectively, which may also be referred to as tilt and pan, respectively. In certain implementations, the system uses an articulating camera 12, as has been previously described. Briefly, in these implementations, the camera 12 articulates to ensure the surgeon can view all possible locations of the robot arms 14, 16 as well as the desired areas of the surgical theater.

As mentioned above, the approximate camera field of view (C) for a given location of the camera is shown in the implementation of FIG. 8A. The camera field of view (C) is about 100 degrees in this implementation, as defined by the angle θ₁ created along the diagonal of the cross section of a rectangle. Any other known field of view angle can be used. It is appreciated that many other angles are possible. In these implementations, it is understood that the surgeon/user is able to view both robot end effectors 18, 20 over a wide range of working distance.

Further, as the robotic device 10 makes large motions with its arms 14, 16 - like those described in FIGS. 5 & 6 - the robot camera tip 12B can be moved using active joints in coordination with the large arm movements to view the entire robot workspace. In certain implementations, the joints of the camera are actively controlled using motors and sensors and a control algorithm implemented on a processor.

The system 1 according to certain implementations has a processor constructed and arranged to execute such a control algorithm. The control algorithm can be provided on computer-readable medium on a processor optionally having an operating system, memory, an input/output interface and the like, as would be appreciated by one of skill in the art. The processor in various implementations can be disposed in the camera handle 12A, device body 10A, in the surgical console 100 or elsewhere, as would be appreciated by those of skill in the art. For purposes of the discussed implementations, the processor is located inside the surgical console 100 as would be readily appreciated.

In these implementations, the control algorithm allows for automated and / or semi-automated positioning and re-positioning of the camera 12 about the pitch (α) and / or yaw (β) rotations shown in FIGS. 8B and 8C, relative to the robotic device 10. This 2 degrees-of-freedom (DOF) system can also be constructed and arranged to translate the camera tip 12B as the robotic device 10 articulates. It is understood that alternative designs are possible.

In the implementation of FIG. 8B and FIG. 8C, the system 1 executes a control algorithm such as an algorithm as discussed above. According to these implementations, the camera 12 is capable of rotating relative to the robot body 10A so as to direct or "point" the camera 12 in various directions to alter the field of view. In this implementation, a robot coordinate frame {R} is affixed to the robot body 10A and is defined by orthogonal unit vectors x_{R}, y_{R}, and z_{R}. A camera coordinate frame {C} is defined with relation to the location of the imaging tip 12B of the camera. In this implementation, the {C} frame is defined by the orthogonal unit vectors xc, yc, and zc, as shown in FIGS. 8B-8D.

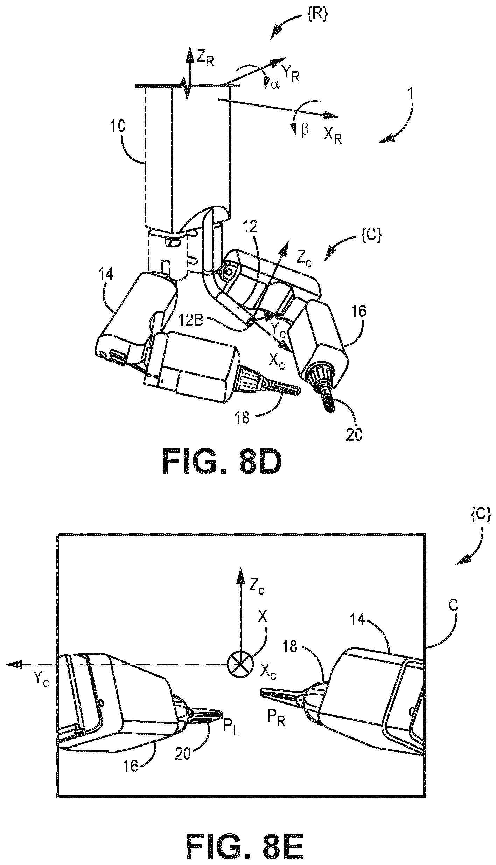

In this implementation, the xc axis is located so as to extend outward from the imaging tip 12B as an extension of the longitudinal axis of the camera 12 and thus point directly in line with the field of view of the camera 12 (as shown in FIG. 8A at C). The yc axis points directly to the left of the camera image and the zc axis is vertical when viewed by the camera imager. The {C} frame is shown from the perspective of the camera in FIG. 8E.

In this implementation, two angles are defined to describe the 2 DOF rotation of the camera frame {C} relative to the robot frame {R}; first angle α and second angle β. Many angles can be used, but in this representative implementation, fixed angles are used and are described by rotations about the x_{R} and y_{R} frames.

The first angle α is defined as a rotation of the camera tip 12B (xc axis) relative to the x_{R} axis about the y_{R} axis, as is shown in FIG. 8B. The second angle β is defined as a rotation of the camera tip 12B (xc axis) relative to the y_{R} axis about the x_{R} axis, as is shown in FIG. 8C.

In these implementations, the system can generate coordinate transformations from one of the camera frame {C} and / or the robot frame {R} to the other - or to any other coordinate frame.

As shown in the implementations of FIGS. 8D-8I, the system 1 according to certain implementations can be constructed and arranged to execute a control algorithm and move the camera 12 and arms 14, 16 in response to the defined camera frame {C} and / or the robot frame {R}. That is, it is understood that in certain implementations, the surgeon or user commands robotic device 10 motion based on images returned by the camera 12, and that the system 1 is constructed and arranged to adjust the locations of various reference frames and components, as described herein.

According to certain of these implementations, the camera frame {C} is fixed to the camera tip 12B so it does not move relative to the view provided by the surgeon.

As shown in FIG. 8E, the system 1 according to these implementations establishes an origin (shown at X) of the camera frame {C} at the intersection of the xc-yc- and zc-axis. Likewise, the robot frame {R} establishes a reference point or origin relative to the position of the device components for coordinated translation between the frames {C}, {R}, as would be understood.

Continuing with the implementation of FIG. 8E, the locations P_{L} and P_{R} of the end effectors 18, 20 can then be located within the camera frame {C}. The location of the end effectors 18, 20 is known in the robot frame {R} as that is what is controlled to operate the robot. Then a coordinate transformation is established between the {R} frame and the {C} frame to locate the position of the end effectors 18, 20 in the camera frame.

It is understood that the positioning of the camera 12 according to these implementations can be controlled and / or planned using several approaches. One approach is to allow the user to control the position of the camera 12 via an input device operably coupled to the console 100, and as described in detail in relation to FIG. 11A and FIG. 11B. Some non-limiting examples of the input device include, for example, a hand or foot controlled joystick. Further implementations have independent joystick-like devices that control the various motions - for example pitch α and yaw β - of the camera. A further approach includes toggling the function of one of the robot hand controllers and / or pedal to then temporarily use the hand controller to command the motion of the camera 12.

In further alternate implementations, additional data relating to the position of the camera 12 and other components such as the arms 14, 16 can be used to establish the reference frames {R}, {C} to choose the direction of the camera 12. These implementations can include end effector 18, 20 positions and velocities as well as many factors associated with the motion of the tools, as would be appreciated by those of skill in the art.

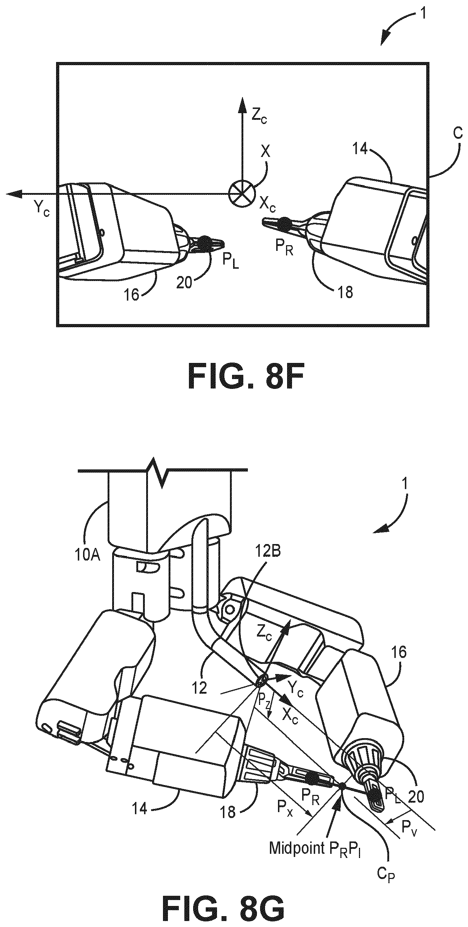

An approach in accordance with the present invention is to control the movement of the camera 12 to be fixed on the end effectors 18, 20. When viewed from the camera perspective C according to these implementations, the end effector 18, 20 locations are defined as P_{L} and P_{R}, where P_{L} and P_{R} are vectors containing the x, y, and z coordinates of the location of the respective points. These can be detected via the camera 12 and their position can be established in the camera frame, as is shown in FIG. 8D, FIG. 8E and FIG. 8F.

In accordance with the present invention, it is therefore possible to calculate the midpoint *Midpoint P_{L}P_{R}* between the end effectors in the camera frame FIG. 8G. In these implementations, a line is created between the left 16 and right 18 end effector locations P_{L} and P_{R}, as is shown in FIG. 8G. The midpoint of that line ^{C}P can then be located in the camera coordinate frame - or in any other frame using known coordinate transformation matricies - where: ${c}_{{P}_{¯}} = Midpoint {{P}_{R} {P}_{L}}_{¯} = \left[\begin{matrix}{P}_{x} \\ {P}_{y} \\ {P}_{z}\end{matrix}\right]$

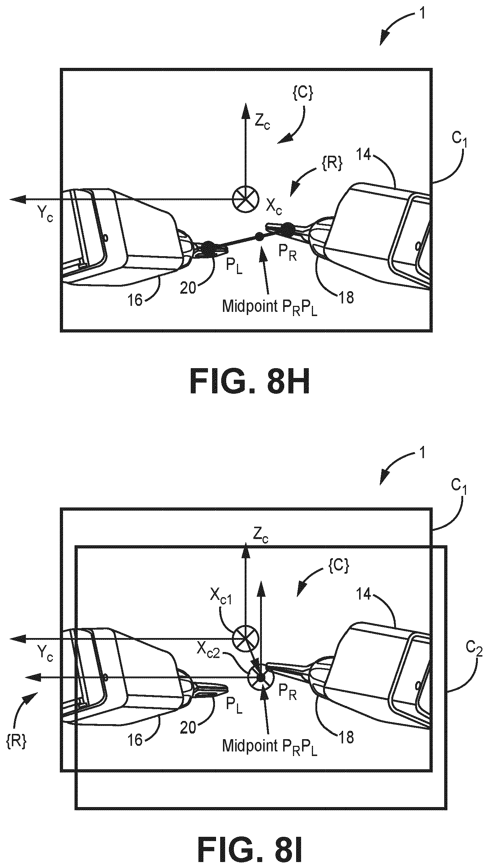

Using these reference frames, it is possible to re-position an initial camera view C₁ to a second camera view C₂ via coordinate transformations to ensure the camera 12 remains centered on the tools 18, 20. For example, as is shown in FIG. 8H, when viewed from the initial camera view C₁, the {R} midpoint *P_{R}P_{L}* can be observed relative to the camera coordinate frame {C}. It is understood that the {C} reference frame origin X_{c} is not aligned with the midpoint *P_{R}P_{L}* established by the {R} reference frame.

The camera 12 can then be re-positioned so as to zero the origin point X_{c} of the camera to the midpoint *P_{R}P_{L}* of the two tools 18, 20 via coordinate transformations, as is shown in FIG. 8I at X_{C1} → X_{C2}. This motion can also be damped. In these implementations, the system 1 retards the motion of the camera tip 12B by reducing the motion of the tip with a term proportional to the velocity of the tip, as would be understood.

Further implementations involving the control of camera 12 utilize a running average position of each right 18 and left 20 end effector is calculated. In these implementations, the difference between average position and actual position is calculated for each arm 14, 16. If the difference is greater than a threshold value, the arm is considered to be moving. In these implementations, camera actuation outputs are calculated via the kinematics of the camera as compared with a target position. When only one arm is moving, the target position is the position of only the moving arm. If both arms are moving, the midpoint between the two end-effector positions is used as the target position, as would be understood.

In implementations such as these running-average kinematic control execute pseudo-code such as:

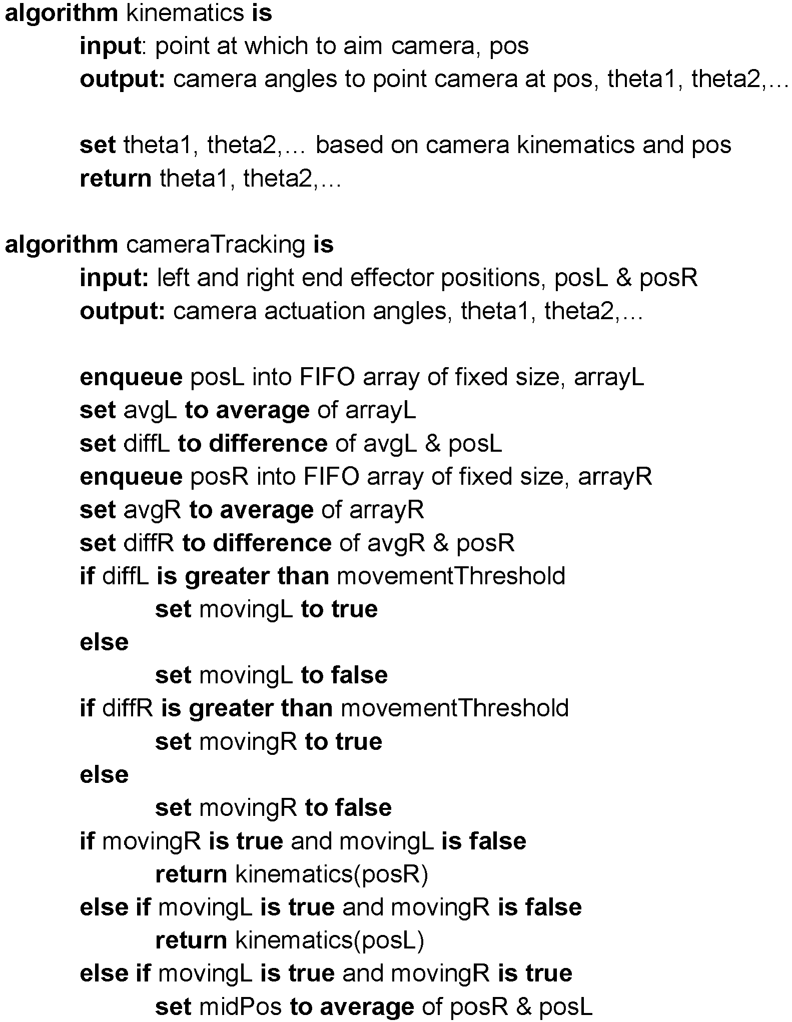

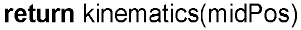

Alternatively, other clinical and robotic factors can be used to determine the camera location. For example, the velocity/position and/or the velocity/position history can be considered in the commanded camera position. In constructing and arranging the system, it is understood that a tool that moves quickly, often, or constantly, or other factors could "pull" the camera toward that tool, and that a more stationary tip may not hold the camera as close.

Further, it is well appreciated that various machine learning techniques or other algorithms can be used to determine the orientation of the camera 12. This could include neural networks, genetic algorithms, or many other machine learning algorithms known and appreciated in the art.

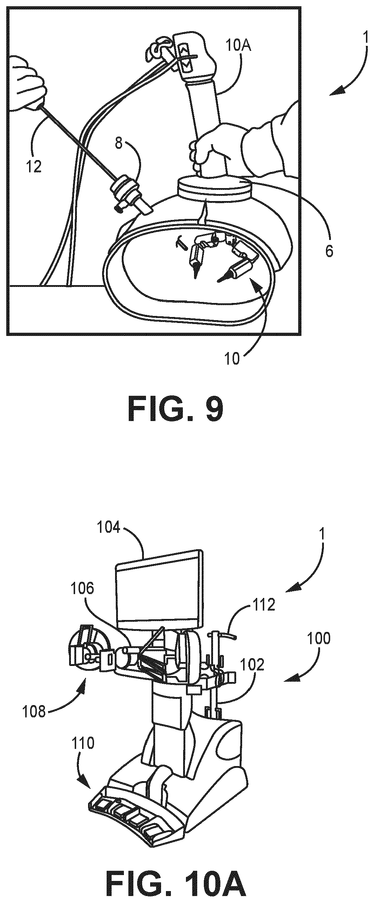

Alternatively, the surgeon may also choose to remove the camera 12 from the robotic device 10 and use it in another, known laparoscopic port 8 like a standard manual laparoscope as shown in FIG. 9. It is understood that this perspective may be useful to visualize the robotic device 10 to ensure safe insertion and extraction via the main port 6. The camera 12 according to these implementations can also be removed from the robotic device 10 so the optics can be cleaned.

In certain implementations, the robotic device is piloted from the surgeon console 100 as shown in FIG. 10A. This exemplary implementation of the surgeon console 100 contains a main computer 102 that performs robot control functions and system monitoring. In these implementations, the surgeon views the surgical environment using the output of the robotically articulated camera shown on a high-definition real-time display 104. Several functions of the console and robot are controlled through a touch screen interface 106. The touch screen 106 is also used to display some information about the state of the robot. Alternatively, any known console can be used with the various implementations of the system disclosed or contemplated herein.

The device 10 and camera 12 motion are controlled in this implementation via the surgeon console 100 with left and right hand input devices 108. The input devices 108 interface with the surgeon's hands and monitor the surgeon's movement. As has been previously described, the input devices 108 have a surgeon presence sensor to indicate the surgeon's hands are properly engaged. The devices can also provide haptic feedback by pushing on the surgeon's hands to indicate things such as workspace boundaries and to prevent collisions between the robot arms, as was also described in the referenced documents. These input devices 108 also control open/close functions of the robot's surgical tools.

The surgeon console 100 according to these implementations can also have foot pedals 110 that are used to control various robot functions including clutching, camera movements, and various electro cautery functions. Alternatively, other input devices on the console can be used to control those various functions.

The surgeon console 100 according to these implementations is constructed and arranged to be used in either a sitting (similar to Intuitive's da Vinci) or standing position (similar to manual laparoscopy). The console 100 is designed to be easily transported between operating rooms using castors and a transport handle 112.

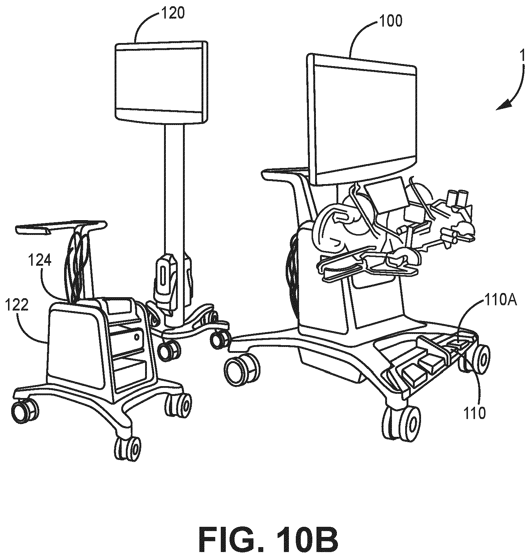

A further implementation of the surgeon console 100 is shown in FIG. 10B. In these implementations, additional, alternative support equipment is provided, here, a remote display 120 and a companion cart 122. It is understood that the space around a patient during a surgery is valuable, and that certain wired or otherwise connected components have limited range.

The remote display 120 according to these implementations is operably coupled to the other components and can be wireless or wired. This display 120 can be used to show the view from the robot camera or any other video.

In the implementation of FIG. 10B, a companion cart 122 is also provided. The cart 122 can be used to hold the robot interface pod 124 or an electro surgical generator or other equipment.

In certain implementations one 110A of the foot pedals 110 or another input device can be used as a clutch that separates coordinated motion of the hand input devices from the motion of the robot. In certain implementations, the foot pedals 110 can be configured allow the user to move the hand input devices 108 to a more desirable location in their own workspace. Then the coordinated motion can be reengaged. Alternatively, in other implementations the clutch function might separate the coordinated motion of the hand input devices from the motion of the robot and then the hand input devices might automatically move to a desired potion. Then the coordinated motion can be reengaged.

In certain system implementations, various cables 126 are used to connect the robot, camera, electrosurgical generator, and the surgeon console, as is shown in FIG. 11A.

According to one implementation, all connections of the cables 126 to and from the various system 1 components are made through a connection pod 124, shown in FIG. 10B, FIG. 11A and FIG. 11B. The cables and connectors are shown schematically in FIG. 11A.

In these implementations, the pod 124 is permanently connected to the surgeon console 100 via an approximately 20' (6 meters) cable 126 giving flexibility in the placement of the surgeon console within the operating room. Other lengths are of course possible. It is understood that in use, the pod 124 and cable 126 can be hung from the back of the console 100 for transport. When in use, the pod 124 can be placed near the electrosurgical generator and / or near the operating table.

In various implementations, the robotic device 10 and camera 12 both have pigtails 126A, 126B that are permanently attached to the robot and camera and then have connectors at the pod. The robot pigtail 126A carries electrical power and control signals as well as cautery energy. The camera pigtail 126B carries electrical power and control signals as well as a fiber optic cable for the video signal.

The pod 124 according to these implementations can also be constructed and arranged to interface with an electrosurgical generator (ESG) 128. On/Off control signals from the user at the surgeon console 100 are directly connected to the ESG 128 control inputs. The mono-polar return pad 130 is first connected to the pod 124 and then the cautery energy is routed from the ESG 128 to the appropriate surgical tools via the pod 124. In various implementations, each connection contains a sensor that allows the surgeon console to determine if connections are made correctly. This system 1 has been designed to ensure safety and simplicity of setup.

One interface pod 124 design is shown in FIG. 11B. In this implementation, the companion cart 122 is used to house the interface pod 124 and ESG 128. The interface pod connects to the surgeon console and the electro surgical unit. The interface pod 124 then has connections for the robotic device 10 and camera 12.

In various implementations, a known, commercially-available ESG 128 can interface with the system, according to one implementation. For example, in one specific implementation, the surgeon console can have two (IPX7) foot pedals 110 that open and close an electrical circuit that activates and deactivates the ESG 128. The pedals 110 are directly connected to the ESG 128. As a safety measure, the surgeon console 100 can disconnect the pedals from the ESG 128, but cannot activate the ESG 128. Activation of the ESG 128 requires the surgeon to also depress the pedals 110. Mono-polar cautery energy is delivered to the right arm of the robot and bi-polar energy is delivered to the left arm. The electrocautery energy is delivered to the surgical target through the specifically designed surgical tools - such as a grasper for bi-polar and scissors and hood for mono-polar energy. Verification testing -creepage, clearance, impedance and the like - has been performed to ensure proper interoperability function between the electrosurgical generator and the system.

Alternatively, the ESG 128 can interface with the system 1 through other input devices other than the foot pedals. Alternatively, the system has no pod 124. In addition to these specialized subsystems, certain implementations of the system can utilize one or more of the many standard general surgical and laparoscopic systems and techniques that are commonly available and provided by the users, as described below.

Further aspects of the system 1 are described herein.

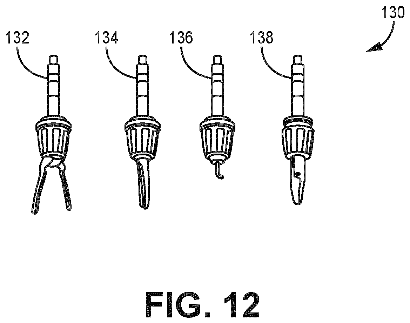

FIG. 12 depicts views of various surgical tools (also referred to above as end effectors 18, 20) are the "hands" of the system and shown generally at 130. Four tools are shown in FIG. 12, including a fenestrated grasper 132 that is capable of bi-polar cautery, a scissors 134 that delivers mono-polar cautery, a hook 136 that delivers mono-polar cautery, and a left/right needle driver set 138. Alternatively, other end effectors can be used with the implementations disclosed or contemplated herein.

In certain implementations, these surgical instruments 130 are designed to be single-use disposable accessories to the robot system 1. They can be chosen based on clinical need for the specific surgical task.

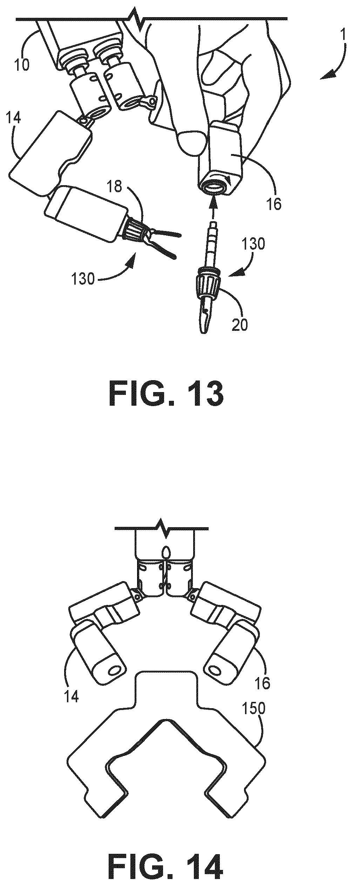

The tools 130 are inserted into the distal end of the robot forearm 14, 16 and then are locked in place using a ¼-turn bayonet-style connection as end effectors 18, 20, as shown in FIG. 13. The tools 130 are removed by reversing the process. When the tools 130 are inserted they interact with connections inside the forearm to deliver cautery energy to the tool tip. Alternatively, any coupling mechanism can be used to couple any of the end effectors with the robotic device.

According to certain implementations, the surgical robotic device 10 is intended to be cleaned and sterilized for reuse. The robotic device 10 has a molded silicon protective sleeve (not shown) that covers the areas between the robot base link and the forearms. This enables the robot to be cleaned and fully exposed during the sterilization process.

In certain implementations, protective and fitted sleeves are provided that are tailored to cover the robot arms 14, 16. One such sleeve 140 is shown in FIG. 14 prior to installation onto the robot arms 14, 16. The sleeve 140 is flexible so it does not restrict motion of the robot arms 14, 16 and is durable to tear and puncture during normal robot operation. The sleeve 140 serves as a barrier to fluid ingress into the robot. It is made of biocompatible material and, like all other tissue contact materials in the system, is compliant with ISO 10993. The robot sleeve 140 can be factory installed and stays on the robot throughout the useful life of the device 10.

The robot sleeve 140 also makes the device easily cleaned post-surgery and ensures that all patient contact surfaces are properly exposed during the sterilization process. Alternatively, any known sleeves or protective components can be used.

In certain implementations, a robot clamp 150 is provided to support the device 10 during the procedure. In these implementations, a known, commercially-available support arm 4 can be used to anchor the device 10 to the operating table 2, as shown in FIG. 15. It is understood that the support arm 4 has several adjustment features so it can provide stability while allowing significant repositioning of the robot. In certain examples, the support arm adjustment features are controlled using one adjustment knob 142.

One clamp 150 implementation is depicted in FIG. 16. The device 10 according to these implementations interfaces with the support arm 4 through a robot clamp 150 as shown in FIG. 16. The clamp 150 has a safety release button 152 that must be pressed prior to clamping or unclamping the device 10. The robot body 10A has a robot clamp interface ring 154 defined in the housing 11 to provide an interface between the clamp 150 and the device 10. After the release button 152 is pressed the robotic device 10 can be inserted or removed from the clamp 150 using the release lever 156.

In implementations such as these, the clamp 150 has a clothespin member 158 that is optionally V-grooved. The clothespin member 158 permits the smooth and controlled rotation of the device 10. In these implementations, a clasping member 160 is disposed opposite the clothespin member 158, which is urged inward to secure the device at the interface ring 154, as would be appreciated.

Although various preferred implementations have been described, persons skilled in the art will recognize that changes may be made in form and detail without departing from the scope thereof.

Although the present invention has been described with reference to preferred implementations persons skilled in the art will recognize that changes may be made in form and detail without departing from the scope of the invention

## Claims

1. A robotic surgical system (1), comprising:
a) a device body (10A) constructed and arranged to be positioned at least partially within a body cavity of a patient through an incision, the device body comprising:
i) a first robotic surgical arm (14) operably coupled to the device body (10A) and comprising a first end effector (18);
ii) a second robotic surgical arm (16) operably coupled to the device body (10A) and comprising a second end effector (20); and
b) a positionable camera (12) operably coupled to the device body (10A), the positionable camera (12) constructed and arranged to provide views of the first and second end effectors (18, 20); and
c) a surgical console (100) comprising a processor constructed and arranged to execute an algorithm to position the positionable camera (12), the algorithm configured to
establish a midpoint P_{L}P_{R} between the first and second end effectors via defined locations P_{L} of the first end effector and P_{R} of the second end effector; and
wherein the processor is configured to establish a robot reference frame and a camera reference frame, the camera reference frame having an origin defined by an intersection of orthogonal unit vectors x_{C}, y_{C}, and z_{C},
wherein the algorithm, when executed, positions the positionable camera (12) such that the origin is aligned with the midpoint P_{L}P_{R}.

2. The robotic surgical system (1) of claim 1 (10D), further comprising a camera lumen (1) defined in the device body (10A).

3. The robotic surgical system of claim 1, wherein the processor is configured to align the camera reference frame with the robot reference frame and re-position the positionable camera (12).

4. The robotic surgical system (1) of claim 1, wherein the positioning of the positionable camera (12) to align with the Midpoint P_{L}P_{R} comprises a damped motion.

5. The robotic surgical system (1) of claim 4, wherein the damped motion of the positionable camera (12) comprises a term proportional to a velocity of a tip of the positionable camera (12).

6. The robotic surgical system of claim 1, wherein the positionable camera (12) comprises a tip configured to extend from a distal end of the device body (10A).

7. The robotic surgical system of claim 6, wherein the tip (12C) of the positionable camera (12) is constructed and arranged to be capable of both pitch and yaw.

8. The robotic surgical system (1) of claim 1, wherein the processor is further configured to execute a control algorithm for positioning of the first and second robotic surgical arms (14, 16).

9. The robotic surgical system of claim 1, wherein the processor is constructed and arranged to execute a control algorithm for positioning of the first and second robotic surgical arms (14, 16), wherein the processor determines running average positions of the first end effector and second end effector (18, 20), and wherein the algorithm to position the positionable camera (12) uses the running average positions of the first end effector (18) and second end effector (20) to align the articulating tip (12C).

10. The robotic surgical system of claim 9, wherein the processor determines whether the first end effector (18) is moving based upon the running average position of the first end effector (18) changing more than a threshold amount, wherein the processor determines whether the second end effector (20) is moving based upon the running average position of the second end effector (20) changing more than the threshold amount, and wherein the algorithm to position the positionable camera (12) is only executed upon a determination that both of the first end effector (18) and the second end effector (20) are moving.

11. The robotic surgical system of claim 9, wherein the processor determines whether the first end effector (18) is moving based upon the running average position of the first end effector (18) changing more than a threshold amount, wherein the processor determines whether the second end effector (20) is moving based upon the running average position of the second end effector (20) changing more than the threshold amount, and wherein the algorithm to position the positionable camera (12) is executed upon a determination that neither the first end effector (18) nor the second end effector (20) is moving.

## Patentansprüche

1. Robotisches chirurgisches System (1), umfassend:
a) einen Vorrichtungskörper (10A), der konstruiert und angeordnet ist, um durch eine Inzision mindestens teilweise innerhalb einer Körperhöhle eines Patienten positioniert zu werden, wobei der Vorrichtungskörper umfasst:
i) einen ersten robotischen chirurgischen Arm (14), der funktionell an den Vorrichtungskörper (10A) gekoppelt ist und einen ersten Endeffektor (18) umfasst;
ii) einen zweiten robotischen chirurgischen Arm (16), der funktionell an den Vorrichtungskörper (10A) gekoppelt ist und einen zweiten Endeffektor (20) umfasst; und
b) eine positionierbare Kamera (12), die funktionell an den Vorrichtungskörper (10A) gekoppelt ist, wobei die positionierbare Kamera (12) konstruiert und angeordnet ist, um Ansichten des ersten und zweiten Endeffektors (18, 20) bereitzustellen; und
c) eine chirurgische Konsole (100), umfassend einen Prozessor, der konstruiert und angeordnet ist, um einen Algorithmus auszuführen, um die positionierbare Kamera (12) zu positionieren, wobei der Algorithmus ausgebildet ist, um
einen Mittelpunkt PLPR zwischen dem ersten und zweiten Endeffektor über vorgegebene Positionen PL des ersten Endeffektors und PR des zweiten Endeffektors zu erstellen; und
wobei der Prozessor ausgebildet ist, um einen Roboterreferenzrahmen und einen Kamerareferenzrahmen zu erstellen, wobei der Kamerareferenzrahmen einen Ursprung aufweist, der durch einen Schnittpunkt der orthogonalen Einheitsvektoren xc, yc und zc definiert ist,
wobei der Algorithmus bei Ausführung die positionierbare Kamera (12) derart positioniert, dass der Ursprung mit dem Mittelpunkt P_{L}P_{R} ausgerichtet ist.

2. Robotisches chirurgisches System (1) nach Anspruch 1 (10D), weiter umfassend ein Kameralumen (1), das in dem Vorrichtungskörper (10A) definiert ist.

3. Robotisches chirurgisches System nach Anspruch 1, wobei der Prozessor ausgebildet ist, um den Kamerareferenzrahmen mit dem Roboterreferenzrahmen auszurichten und die positionierbare Kamera (12) neu zu positionieren.

4. Robotisches chirurgisches System (1) nach Anspruch 1, wobei das Positionieren der positionierbaren Kamera (12) zum Ausrichten mit dem Mittelpunkt P_{L}P_{R} eine gedämpfte Bewegung umfasst.

5. Robotisches chirurgisches System (1) nach Anspruch 4, wobei die gedämpfte Bewegung der positionierbaren Kamera (12) einen Term umfasst, der proportional zu einer Geschwindigkeit einer Spitze der positionierbaren Kamera (12) ist.

6. Robotisches chirurgisches System nach Anspruch 1, wobei die positionierbare Kamera (12) eine Spitze umfasst, die ausgebildet ist, um sich von einem distalen Ende des Vorrichtungskörpers (10A) zu erstrecken.

7. Robotisches chirurgisches System nach Anspruch 6, wobei die Spitze (12C) der positionierbaren Kamera (12) so konstruiert und angeordnet ist, dass sie sowohl zu Nickals auch Gierbewegungen fähig ist.

8. Robotisches chirurgisches System (1) nach Anspruch 1, wobei der Prozessor weiter ausgebildet ist, um einen Steueralgorithmus zum Positionieren des ersten und zweiten robotischen chirurgischen Arms (14, 16) auszuführen.

9. Robotisches chirurgisches System nach Anspruch 1, wobei der Prozessor konstruiert und angeordnet ist, um einen Steueralgorithmus zum Positionieren des ersten und zweiten robotischen chirurgischen Arms (14, 16) auszuführen, wobei der Prozessor gleitende Durchschnittspositionen des ersten Endeffektors und zweiten Endeffektors (18, 20) bestimmt, und wobei der Algorithmus zum Positionieren der positionierbaren Kamera (12) die gleitenden Durchschnittspositionen des ersten Endeffektors (18) und des zweiten Endeffektors (20) zum Ausrichten der Gelenkspitze (12C) verwendet.

10. Robotisches chirurgisches System nach Anspruch 9, wobei der Prozessor basierend darauf, dass sich die gleitende Durchschnittsposition des ersten Endeffektors (18) um mehr als einen Schwellenwertbetrag ändert, bestimmt, ob sich der erste Endeffektor (18) bewegt, wobei der Prozessor basierend darauf, dass sich die gleitende Durchschnittsposition des zweiten Endeffektors (20) um mehr als den Schwellenwertbetrag ändert, bestimmt, ob sich der zweite Endeffektor (20) bewegt, und wobei der Algorithmus zum Positionieren der positionierbaren Kamera (12) nur bei einer Bestimmung ausgeführt wird, dass sich sowohl der erste Endeffektor (18) als auch der zweite Endeffektor (20) bewegt.

11. Robotisches chirurgisches System nach Anspruch 9, wobei der Prozessor basierend darauf, dass sich die gleitende Durchschnittsposition des ersten Endeffektors (18) um mehr als einen Schwellenwertbetrag ändert, bestimmt, ob sich der erste Endeffektor (18) bewegt, wobei der Prozessor basierend darauf, dass sich die gleitende Durchschnittsposition des zweiten Endeffektors (20) um mehr als den Schwellenwertbetrag ändert, bestimmt, ob sich der zweite Endeffektor (20) bewegt, und wobei der Algorithmus zum Positionieren der positionierbaren Kamera (12) bei einer Bestimmung ausgeführt wird, dass sich weder der erste Endeffektor (18) noch der zweite Endeffektor (20) bewegt.

## Revendications

1. Système chirurgical robotisé (1), comprenant :
a) un corps de dispositif (10A) construit et agencé pour être positionné au moins partiellement dans une cavité corporelle d'un patient à travers une incision, le corps de dispositif comprenant :
i) un premier bras chirurgical robotisé (14) couplé de manière opérationnelle au corps de dispositif (10A) et comprenant un premier effecteur terminal (18) ;
ii) un second bras chirurgical robotisé (16) couplé de manière opérationnelle au corps de dispositif (10A) et comprenant un second effecteur terminal (20) ; et
b) une caméra positionnable (12) couplée de manière opérationnelle au corps de dispositif (10A), la caméra positionnable (12) étant construite et agencée pour fournir des vues des premier et second effecteurs terminaux (18, 20) ; et
c) une console chirurgicale (100) comprenant un processeur construit et agencé pour exécuter un algorithme afin de positionner la caméra positionnable (12), l'algorithme étant configuré pour
établir un point médian P_{L}P_{R} entre les premier et second effecteurs terminaux via des emplacements définis P_{L} du premier effecteur terminal et PR du second effecteur terminal ; et
dans lequel le processeur est configuré pour établir un cadre de référence de robot et un cadre de référence de caméra, le cadre de référence de caméra présentant une origine définie par une intersection de vecteurs d'unité orthogonaux xc, yc et zc,
dans lequel l'algorithme, lorsqu'il est exécuté, positionne la caméra positionnable (12) de telle sorte que l'origine soit alignée avec le point médian P_{L}P_{R}.

2. Système chirurgical robotisé (1) selon la revendication 1 (10D), comprenant en outre une lumière de caméra (1) définie dans le corps de dispositif (10A).

3. Système chirurgical robotisé selon la revendication 1, dans lequel le processeur est configuré pour aligner le cadre de référence de caméra avec le cadre de référence de robot et repositionner la caméra positionnable (12).

4. Système chirurgical robotisé (1) selon la revendication 1, dans lequel le positionnement de la caméra positionnable (12) pour s'aligner avec le point médian P_{L}P_{R} comprend un mouvement amorti.

5. Système chirurgical robotisé (1) selon la revendication 4, dans lequel le mouvement amorti de la caméra positionnable (12) comprend un terme proportionnel à une vitesse d'une pointe de la caméra positionnable (12).

6. Système chirurgical robotisé selon la revendication 1, dans lequel la caméra positionnable (12) comprend une pointe configurée pour s'étendre depuis une extrémité distale du corps de dispositif (10A).

7. Système chirurgical robotisé selon la revendication 6, dans lequel la pointe (12C) de la caméra positionnable (12) est construite et agencée pour être capable à la fois de tangage et de lacet.

8. Système chirurgical robotisé (1) selon la revendication 1, dans lequel le processeur est en outre configuré pour exécuter un algorithme de commande pour le positionnement des premier et second bras chirurgicaux robotisés (14, 16).

9. Système chirurgical robotisé selon la revendication 1, dans lequel le processeur est construit et agencé pour exécuter un algorithme de commande pour le positionnement des premier et second bras chirurgicaux robotisés (14, 16), dans lequel le processeur détermine des positions moyennes courantes du premier effecteur terminal et du second effecteur terminal (18, 20), et dans lequel l'algorithme pour positionner la caméra positionnable (12) utilise les positions moyennes courantes du premier effecteur terminal (18) et du second effecteur terminal (20) pour aligner la pointe articulée (12C).

10. Système chirurgical robotisé selon la revendication 9, dans lequel le processeur détermine si le premier effecteur terminal (18) est en mouvement sur la base de la position moyenne courante du premier effecteur terminal (18) qui varie au-delà d'un seuil, dans lequel le processeur détermine si le second effecteur terminal (20) est en mouvement sur la base de la position moyenne courante du second effecteur terminal (20) qui varie au-delà d'un seuil, et dans lequel l'algorithme pour positionner la caméra positionnable (12) n'est exécuté que lorsqu'il est déterminé que le premier effecteur terminal (18) et le second effecteur terminal (20) sont tous deux en mouvement.

11. Système chirurgical robotisé selon la revendication 9, dans lequel le processeur détermine si le premier effecteur terminal (18) est en mouvement sur la base de la position moyenne courante du premier effecteur terminal (18) qui varie au-delà d'un seuil, dans lequel le processeur détermine si le second effecteur terminal (20) est en mouvement sur la base de la position moyenne courante du second effecteur terminal (20) qui varie au-delà d'un seuil, et dans lequel l'algorithme pour positionner la caméra positionnable (12) est exécuté lors d'une détermination selon laquelle ni le premier effecteur terminal (18) ni le second effecteur terminal (20) n'est en mouvement.
